(19) Europäisches Patentamt
European Patent Office
Office européen des brevets

(11) **EP 4 520 254 A1**

(12) **EUROPEAN PATENT APPLICATION**
published in accordance with Art. 153(4) EPC

(43) Date of publication:
**12.03.2025 Bulletin 2025/11**

(21) Application number: **22954118.0**

(22) Date of filing: **11.11.2022**

(51) International Patent Classification (IPC):
***A61B 5/00*** (2006.01)   ***G06T 7/00*** (2017.01)
***G06T 7/60*** (2017.01)

(52) Cooperative Patent Classification (CPC):
**A61B 5/00; G06T 7/00; G06T 7/60**

(86) International application number:
**PCT/KR2022/017759**

(87) International publication number:
**WO 2024/029663 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **04.08.2022 KR 20220097351**

(71) Applicant: **Ahn, Ga Ram
Seoul 06909 (KR)**

(72) Inventor: **Ahn, Ga Ram
Seoul 06909 (KR)**

(74) Representative: **Keilitz Haines & Partner
Patentanwälte PartGmbB
Triftstraße 5
80538 München (DE)**

(54) **SKIN PROCEDURE GUIDING AND FEEDBACK SYSTEM**

(57)    Provided is a skin procedure guidance and feedback system that derives displacements of points before and after formation of a coagulation spot at each point on the skin surface based on an energy-based medical device, and calculates and feeds back a minimum distance that prevents coagulation zones from overlapping when coagulation spots are generated several times based thereon, including a photographing unit that photographs skin before and after the procedure, a displacement calculator that derives landmark displacement data from landmark location data of the skin photographed by the photographing unit, and derives location data of a contraction center based on the landmark displacement data, a boundary deriver that derives a boundary of a contraction zone from the contraction center in the skin procedure, and a procedure feedback unit that takes result data derived by the boundary deriver as input to feed back optimal parameter information for the procedure.

FIG. 1

## Description

[Technical Field]

The present invention relates to a skin procedure guidance and feedback system, and more particularly to a skin procedure guidance and feedback system configured to derive displacements of points before and after formation of a coagulation spot at each point on the skin surface based on an energy-based medical device, and to calculate and feed back a minimum distance that prevents coagulation zones from overlapping when coagulation spots are generated several times based thereon.

[Background Art]

The number of consumers visiting dermatologists due to skin sagging and undergoing skin procedures, such as lifting procedures, has been continuously increasing since such skin procedures have relatively short recovery periods compared to plastic surgeries and effectively enable facial wrinkle lifting (that is, skin area contraction).

Here, an energy-based medical device for skin area contraction forms a coagulation zone on the reticular dermis around a target point. In more detail, the reticular dermis includes spiral collagen bundles, which are arranged in a direction parallel to the skin surface, and when each bundle is thermally coagulated, a spiral structure unwinds and becomes tangled to be shorter. When energy is applied to one point of a reticular dermis layer to form a coagulation spot, the reticular dermis layer within a range of coagulation around the point contracts in a direction toward the coagulation spot in a plane parallel to the skin surface. When several such coagulation spots are formed with a narrow range, immediate contraction of the skin area is observed. Therefore, the energy-based medical device uses this principle to induce a cosmetic effect by flattening the skin.

However, there is a limitation that the effect is not constant when the procedure is performed on actual patients. In other words, efficiency of planar contraction of the skin through induction of multiple dermal coagulation spots is not consistent, and thus the efficiency of linear plane contraction is reduced. In addition, there is the case where efficiency of linear plane contraction is reduced due to overlap between coagulation zones, and when temperature excessively rises (for example, to about 80 degrees Celsius), there is a risk of covalent bonds inside collagen molecules breaking down, causing dissolution thereof.

Therefore, in theory, for optimal plane contraction, there is need for technology for generating a coagulation spot so that adjacent contraction zones are in contact. However, at present, in this procedure, a distance between coagulation spots and output adjustment of the device depend on intuition of an operator, and thus there are limitations in terms of efficiency, reproducibility, and safety of the procedure. Further, since an aspect of skin contraction induced by formation of dermal coagulation spots varies depending on skin conditions of the patients and various factors, there is a problem that efficiency is not consistent every time the procedure is performed.

In addition, a distance between multiple coagulation spots in an energy-based medical device is arbitrarily fixed, and even when a procedure distance can be changed by changing a set value of the device or replacing a handpiece, a determination on usage of a distance value depends on experience and intuition of the operator. In addition, when adjustment of the distance between multiple coagulation spots is limited, efforts are made to increase contraction efficiency by adjusting output of the energy-based medical device. However, this adjustment is made intuitively, and thus there are limitations in terms of efficiency, reproducibility, safety, etc.

[Disclosure]

[Technical Problem]

Therefore, the present invention has been made in view of the above problems, and it is an object of the present invention to provide a skin procedure guidance and feedback system configured to acquire skin surface information before and after formation of a dermal coagulation spot of the skin through an energy-based medical device, and provide feedback to optimize a minimum distance that prevents coagulation zones from overlapping and an output energy factor of the energy-based medical device.

[Technical Solution]

In accordance with the present invention, the above and other objects can be accomplished by the provision of a skin procedure guidance and feedback system including a photographing unit configured to photograph skin before and after a procedure by an energy-based medical device, a displacement calculator configured to derive landmark displacement data from landmark location data of the skin photographed by the photographing unit, and to derive location data of a contraction center O based on the landmark displacement data, a boundary deriver configured to derive a boundary of a contraction zone from the contraction center O in the skin procedure, and a procedure feedback unit configured to take result data derived by the boundary deriver as input to feed back optimal parameter information for the procedure.

The energy-based medical device may be configured to apply energy to one focus of the skin to cause a local temperature rise, thereby generating a contraction zone based on the one focus of the skin, and include high-frequency, laser, and high-intensity focused ultrasound (HIFU) devices.

The photographing unit may photograph a skin surface before the procedure and a skin surface after the procedure based on the energy-based medical device.

Landmarks of the skin may include dots or lines marked with pigment, and sweat glands, hair glands, sebaceous glands, pigmented lesions, moles, skin tumors, blood vessels, and wrinkle lines on a skin surface as reference points enabling tracking of positional change of a skin procedure target part before and after the procedure in the photographed images before and after the skin procedure through the energy-based medical device.

The displacement calculator may include a preprocessor configured to convert location data A of landmarks before the skin procedure on a skin surface in an image of the skin surface before the skin procedure into $A[(i, j)ij]$, and to convert location data B of landmarks after the skin procedure on the skin surface in an image of the skin surface after the procedure into $B[(i', j')ij]$, and a displacement data calculator configured to calculate, as the landmark displacement data D, a displacement vector reflecting a result of movement from location data before the skin procedure to location data after the procedure based on the landmark location data of the skin derived by the preprocessor.

The displacement data calculator may represent the landmark displacement data D as $[(i' - i, j' - j)ij]$.

(Here, $i'$ and $j'$ are a row $i'$ and a column $j'$ of $B[(i', j')ij]$, and $i$ and $j$ are a row $i$ and a column $j$ of $A[(i, j)ij]$.)

The boundary deriver may include a graph deriver configured to derive a displacement graph of each point moving toward the contraction center O for each location on a straight line passing through, at an angle , the contraction center O corresponding to a location of a focus to which the energy-based medical device applies energy based on the landmark displacement data, and a boundary deriver configured to calculate a distance R from the contraction center O to the boundary of the contraction zone based on graph analysis result data derived by the graph deriver.

The graph deriver may set the location data of the contraction center O corresponding to a location of a focus to which the energy-based medical device applies energy based on the landmark displacement data D, the location data of the contraction center O may be a location of a point to which landmark points on a skin surface are commonly directed during skin coagulation according to the procedure, and a location of an intersection point of straight lines, which are obtained by points corresponding to respective elements $(i, j)ij$ of location data A of landmarks before the skin procedure on the skin surface each directed in a direction of an element $ij$ vector of the landmark displacement data D, may be set to $(io, jo)$.

The graph deriver may perform parallel translation by $-io$ on an $i$-axis and $-jo$ on a $j$-axis with respect to a contraction center $O (io, jo, 0)$ in the skin procedure, and perform rotation by the angle at which the contraction center O in the skin procedure is passed through on a $k$-axis, thereby deriving a graph of $k$ with respect to $i$ in an $ik$-plane $(j = 0)$.

The boundary deriver may be configured to derive a graph of a regression curve of a distance by which each point moves toward the contraction center O in the skin procedure for each location on a horizontal line ($= 0$) passing through the contraction center O in the skin procedure, and calculate a distance from the contraction center O to the boundary R of the contraction zone from the photographed image before the procedure based on a fact that the distance is close to a distance from the contraction center O to a point where a slope of a tangent becomes 0 on the regression curve.

The procedure feedback unit may include a simultaneous energy feedback unit configured to feed back a minimum distance D preventing coagulation zones from overlapping each other when coagulation spots are simultaneously generated through the energy-based medical device (simultaneous model).

When a size of the contraction zone derived by the boundary deriver is smaller than a fixed procedure distance of the energy-based medical device, the simultaneous energy feedback unit may feed back high output energy value information to the energy-based medical device so that output of the energy-based medical device is allowed to be increased.

When a size of the contraction zone derived by the boundary deriver is greater than a fixed procedure distance of the energy-based medical device, the simultaneous energy feedback unit may feed back low output energy value information to the energy-based medical device so that output of the energy-based medical device is allowed to be decreased.

The procedure feedback unit may include a sequential energy feedback unit configured to feed back a minimum distance D' that prevents coagulation zones in (N-1)th to Nth rounds from overlapping when coagulation spots on the skin are sequentially generated through the energy-based medical device (sequential model).

The sequential energy feedback unit may feed back a location of a coagulation spot to be generated in the Nth round based on a displacement graph derived by the boundary deriver and information on a size and shape of a contraction zone based on one coagulation spot generated in the (N-1)th round.

[Advantageous effects]

According to the skin procedure guidance and feedback system described above:

First, it is possible to derive displacement of each point on a skin surface that contracts when one focal point of skin is treated using an energy-based medical device.

Second, it is possible to derive a location of a contraction center caused by the procedure based on photographs taken before and after the procedure.

Third, it is possible to quantitatively express an aspect of skin contraction by the procedure based on the derived data.

Fourth, it is possible to quantitatively express an aspect of skin contraction, which varies according to a patient undergoing the procedure and a part thereof and according to a type and output of the device, whenever the system is applied.

Fifth, it is possible to generate data necessary for deriving an optimal next procedure location or procedure output during the procedure based on mathematical calculation rather than intuition of the operator.

Sixth, it is possible to combine distribution information on a contraction aspect of skin in several parts in one procedure target to calculate and propose a two-dimensional (2D) arrangement and order of several procedure points for realizing a change to desired appearance through manipulation of a location of each point on the skin.

[Description of Drawings]

FIG. 1 is a configuration diagram of the present invention;

FIGs. 2 and 3 are schematic diagrams of skin landmarks, a helical structure diagram of collagen in skin, and exemplary views of contraction when a coagulation zone is generated in the skin;

FIG. 4 is a schematic diagram of a skin contraction zone generated by a coagulation range of a reticular dermis layer of the skin when energy is irradiated to the skin using the energy-based medical device;

FIGs. 5 and 6 are diagrams describing that safety and area reduction efficiency may vary depending on the distance between adjacent coagulation spots when reducing the area of the skin by generating several coagulation spots;

FIGs. 7 and 8 are a configuration diagram of a displacement calculator 200 and a process diagram of calculating location data by a preprocessor 200a according to an embodiment of the present invention;

FIG. 9 is a process diagram of calculating displacement data D by a displacement data calculator 200b according to an embodiment of the present invention;

FIG. 10 is a configuration diagram of a boundary deriver 300 according to an embodiment of the present invention;

FIG. 11 is a process diagram of calculating a location of a contraction center O by a graph deriver 300a according to an embodiment of the present invention;

FIG. 12 is a graph of the graph deriver 300a according to an embodiment of the present invention;

FIG. 13 is a process diagram of calculating a size and shape of a coagulation center contraction zone by a boundary deriver 300b according to an embodiment of the present invention; and

FIG. 14 is a calculation process diagram when an angle of a straight line passing through a contraction center is 0 ($\theta$ = 0) according to an embodiment of the present invention; and

FIG. 15 is a configuration diagram of a procedure feedback unit 400 according to an embodiment of the present invention.

[Best Mode]

A skin procedure guidance and feedback system according to embodiments of the present invention will be described in detail with reference to the accompanying drawings. Since the present invention may undergo various changes and have various forms, specific embodiments will be illustrated in the drawings and described in detail in the text. However, this is not intended to limit the present invention to a specific form disclosed, and should be understood to include all modifications, equivalents, or substitutes included in the spirit and scope of the present invention. Like reference numerals have been used for like elements throughout the description of each figure. In the accompanying drawings, dimensions of structures are illustrated to be larger than actual ones for clarity of the present invention, or reduced compared to actual ones for understanding of schematic configurations.

In addition, even though terms such as first and second may be used to describe various components, the components should not be limited by the terms. The terms are only used for the purpose of distinguishing one component from another. For example, a first component may be referred to as a second component, and similarly, the second component may be may be referred to as the first component, without departing from the scope of the present invention. Meanwhile, unless defined otherwise, all terms used herein, including technical or scientific terms, have the same meaning as commonly understood by a person of ordinary skill in the art to which the present invention pertains. Terms such as those defined in commonly used dictionaries should be interpreted as having meanings consistent with meanings in the context of the related art, and should not be interpreted as having ideal or excessively formal meanings unless explicitly defined in the present application.

The present invention provides a skin procedure guidance and feedback system configured to acquire skin surface information before and after formation of a dermal coagulation spot of the skin through an energy-based medical device, and provide feedback to optimize a minimum distance that prevents coagulation zones from overlapping and an output energy factor of the energy-based medical device.

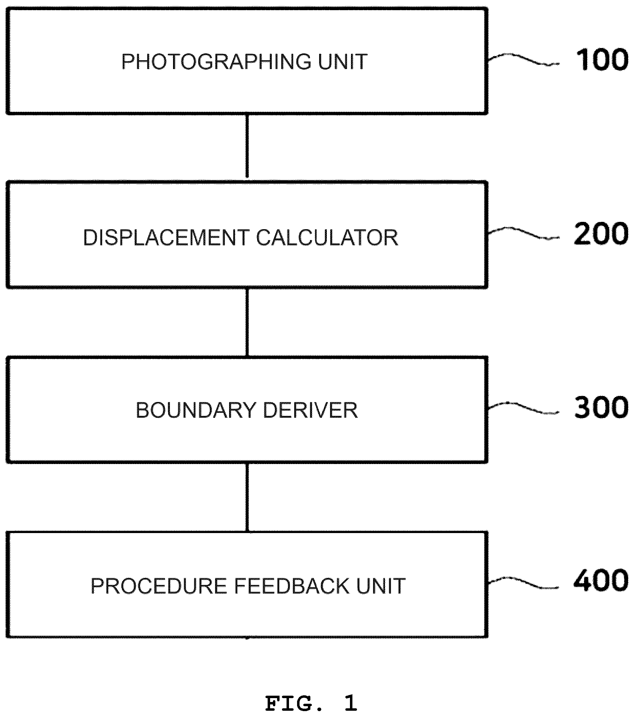

FIG. 1 is a configuration diagram of the present invention.

Referring to FIG. 1, a skin procedure guidance and feedback system includes a photographing unit 100, a displacement calculator 200, a boundary deriver 300, and a procedure feedback unit 400. The photographing unit 100 photographs the skin before and after a procedure by an energy-based medical device, and the displacement calculator 200 derives landmark displacement data from landmark location data of the skin photographed by the photographing unit 100, and derives location data of a contraction center O based on the landmark displacement data. The boundary deriver 300 derives a boundary of a contraction zone from the contraction center O in the skin procedure, and the procedure feedback unit 400 takes result data derived by the boundary deriver 300 as input to feed back optimal parameter information for the procedure.

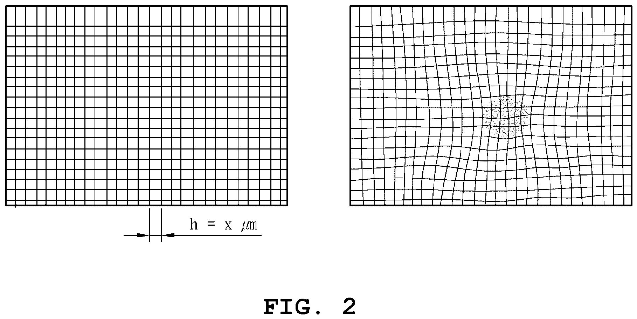

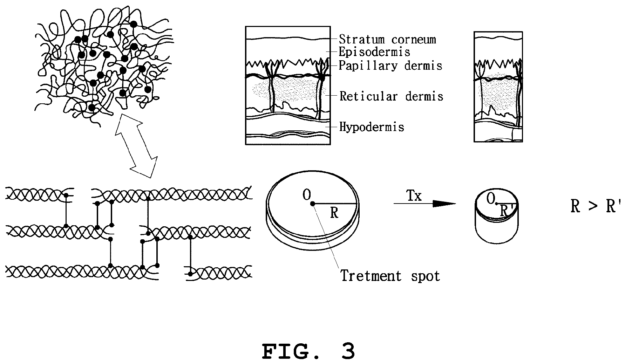

FIGs. 2 and 3 are schematic diagrams of skin landmarks, a helical structure diagram of collagen in skin, and exemplary views of contraction when a coagulation zone is generated in the skin.

Referring to FIG. 2, the skin landmarks are reference points enabling tracking of positional change of a skin procedure target part before and after the procedure in captured images before and after the skin procedure through the energy-based medical device, and include dots or lines marked with pigment, and sweat glands, hair glands, sebaceous glands, pigmented lesions, moles, skin tumors, blood vessels, and wrinkle lines on a skin surface. Referring to FIG. 3, there is a reticular dermis layer filled with collagen bundles in a deep layer of the skin surface. The reticular dermis layer includes spiral collagen bundles, and these collagen bundles are arranged in a direction parallel to the skin surface. When each of the bundles is thermally solidified, the bundle becomes shorter as a spiral structure unwinds and tangles. When a coagulation spot is formed by applying energy to one point of the reticular dermis layer, the reticular dermis layer within a range of coagulation around the point shrinks in a direction toward the corresponding coagulation spot in a plane parallel to the skin surface. Further, a contraction zone caused by one coagulation spot varies depending on several factors such as a patient undergoing the procedure, a part thereof, output of the device, a depth, and an angle.

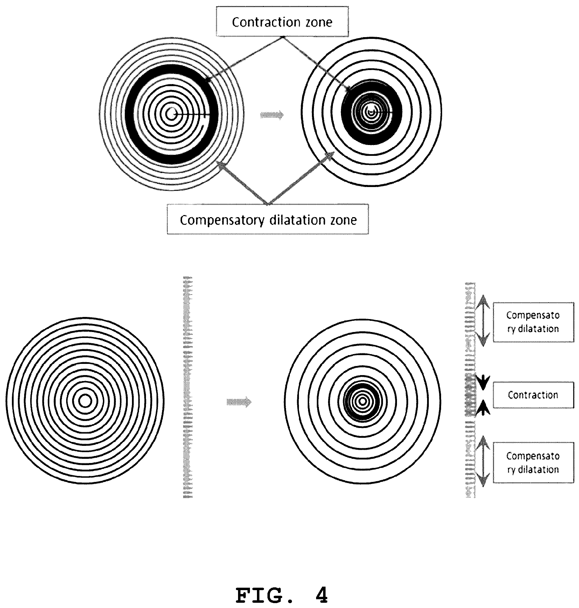

FIG. 4 is a schematic diagram of a skin contraction zone generated by a coagulation range of the reticular dermis layer of the skin when energy is irradiated to the skin using the energy-based medical device.

Referring to FIG. 4, when energy is applied to one focus of the reticular dermis by the energy-based medical device to form a coagulation spot, a contraction zone is formed within a distance R that may vary depending on the angle based on the contraction center O. Within the contraction zone, each point on the skin surface moves toward the contraction center O, and as the density of the points increases, a distance between adjacent points becomes shorter. In addition, as the contraction zone is generated, the distance R from the contraction center O to the contraction zone boundary becomes shorter (R' < R). Conversely, at each point outside the distance R, that is, at a point outside the contraction zone, as the tension increases in a direction toward the contraction center O due to contraction generated within the contraction zone, a compensatory dilatation zone in which the density of each point on the skin surface is lowered is generated.

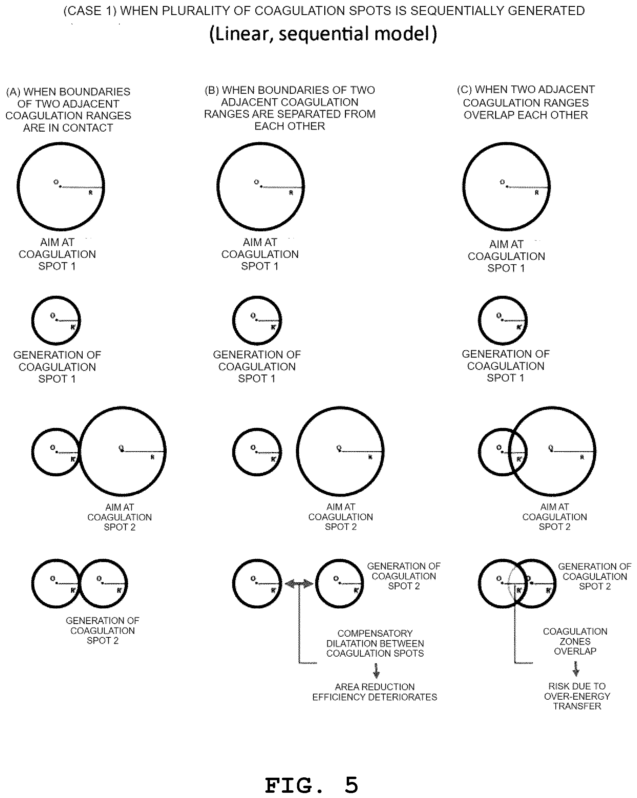

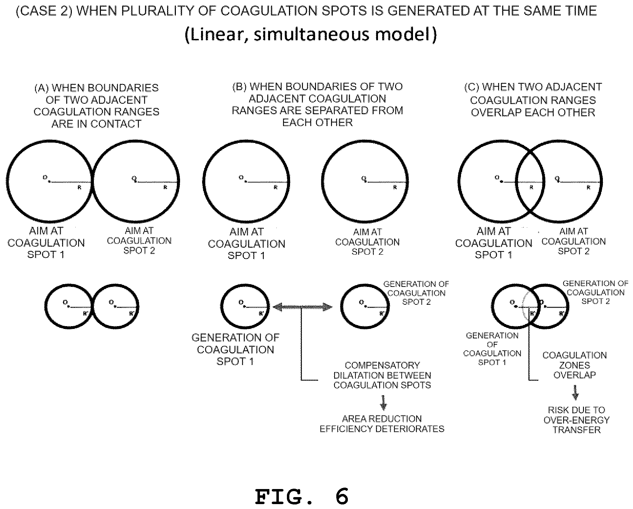

FIGs. 5 and 6 describe that safety and area reduction efficiency may vary depending on the distance between adjacent coagulation spots when reducing the area of the skin by generating several coagulation spots.

Referring to FIGs. 5 and 6, when coagulation ranges are not in contact with each other and are separated from each other, non-coagulated skin therebetween increases (compensatory dilatation), and thus the overall area reduction effect is reduced. Conversely, when overlapping between coagulation zones occurs, there is a risk that temperature may excessively rise (for example, about 80 degrees Celsius or more) and covalent bonds inside collagen molecules may be broken down, causing dissolution thereof.

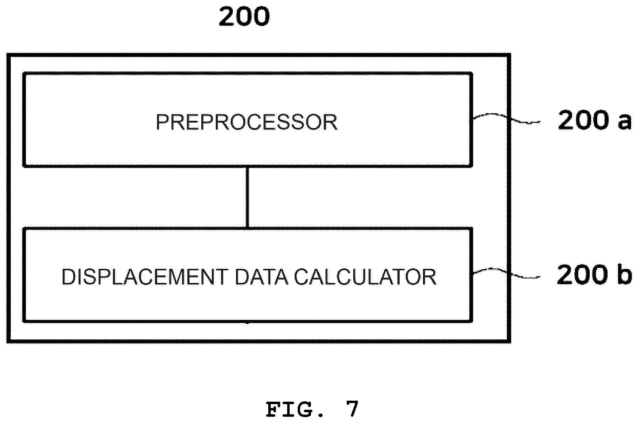

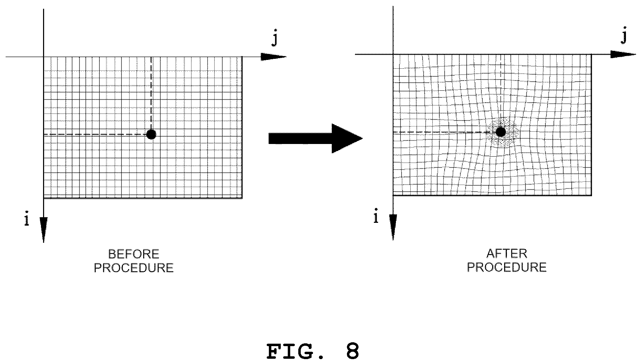

FIGs. 7 and 8 are a configuration diagram of the displacement calculator 200 and a process diagram of calculating location data by a preprocessor 200a according to an embodiment of the present invention.

Referring to FIG. 7, the displacement calculator 200 includes the preprocessor 200a and a displacement data calculator 200b. The preprocessor 200a converts location data A of landmarks before the skin procedure on the skin surface in a captured skin surface image before the procedure into A[(i, j)ij], and converts location data B of landmarks after the skin procedure on the skin surface in a skin surface image after the procedure into B[(i', j')ij]. Further, the displacement data calculator 200b generates, as the landmark displacement data D, a displacement vector reflecting a result of movement from location data before the skin procedure to location data after the procedure based on the location data of the landmarks on the skin derived by the preprocessor 200a.

A more detailed description will be given with reference to FIG. 8. The preprocessor 200a calculates and stores location data A of landmarks on the skin surface before the skin procedure from the image captured before the skin procedure, and calculates and stores location data B of landmarks on the skin surface after the skin procedure from the image captured after the skin procedure according to movement of locations of the landmarks on the skin surface before the skin procedure to the contraction center O due to the skin procedure. Thereafter, the location data A of the landmarks on the skin surface before the skin procedure is represented as A[ (i, j)ij], and the location data B of the landmarks on the skin surface after the skin procedure is represented as B[(i', j')ij]. That is, in more detail, as an embodiment of the present invention, in order to calculate each of the location data A of the landmarks on the skin surface before the skin procedure and the location data B of the landmarks on the skin surface after the skin procedure, pixel coordinates in the captured images before and after the skin procedure are expressed in a 2D Cartesian coordinate system having the origin at a top left vertex of the images, an i-axis directed downward along a vertical line, and a j-axis directed rightward along a horizontal line, and represented by the following matrix A in which coordinates (i, j) of each pixel on the image data correspond to an element in a row i and a column j.

$$
A = \begin{bmatrix} (1,1) & (1,2) & (1,3) & \\ (2,1) & (2,2) & (2,3) & \cdots \\ (3,1) & (3,2) & (3,3) & \\ & \vdots & & \ddots \end{bmatrix}
$$

The location data B of the landmarks on the skin surface after the skin procedure may be represented by a matrix [(i', j')ij] having, as an element, coordinates (i', j') of a point at which each element [(i, j)ij] of the matrix A arrives after contraction.

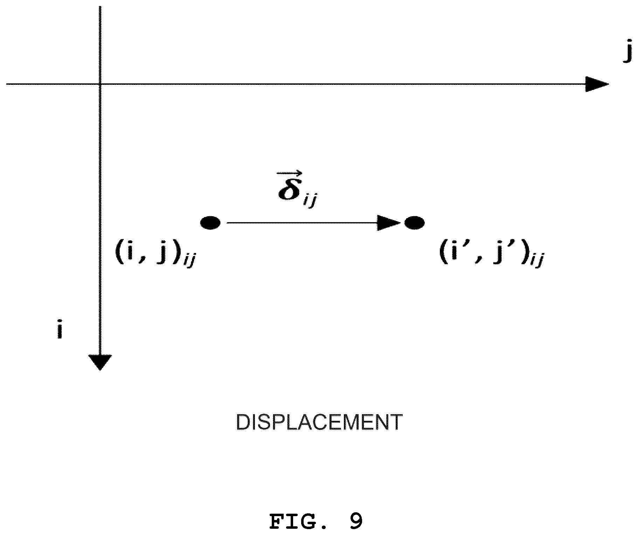

FIG. 9 is a state of calculating the displacement data D by the displacement data calculator 200b according to an embodiment of the present invention.

Referring to FIG. 9, the displacement data calculator 200b represents the landmark displacement data D as [(i' - i, j' - j)ij]. That is, in more detail, the displacement data calculator 200b represents a result obtained by movement of the location data of the skin landmarks derived by the preprocessor from the location data A before the skin procedure to the location data after the procedure by displacement data D expressed as a vector iij. Here, as an example of the displacement data D, D = [(i' - i, j' - j)ij] = [iij].

(Here, i' and j' are a row i' and a column j' of B[(i', j')ij], and i and j are a row i and a column j of A[(i, j)ij].

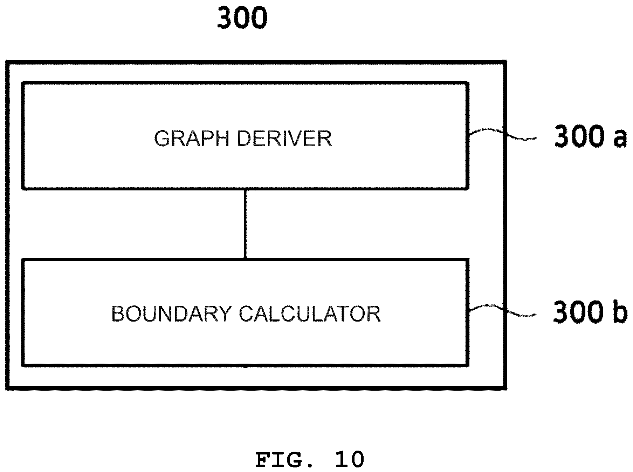

FIG. 10 is a configuration diagram of the boundary deriver 300 according to an embodiment of the present invention.

Referring to FIG. 10, the boundary deriver 300 includes a graph deriver 300a and a boundary deriver 300b. Here, the graph deriver 300a derives a displacement graph of each point moving toward the contraction center O for each location on a straight line passing, at an angle $\ell$, through the contraction center O corresponding to a location of a focus to which the energy-based medical device applies energy based on the landmark displacement data, and the boundary deriver 300b calculates the distance R from the contraction center O to the contraction zone boundary based on graph analysis result data derived by the graph deriver 300a.

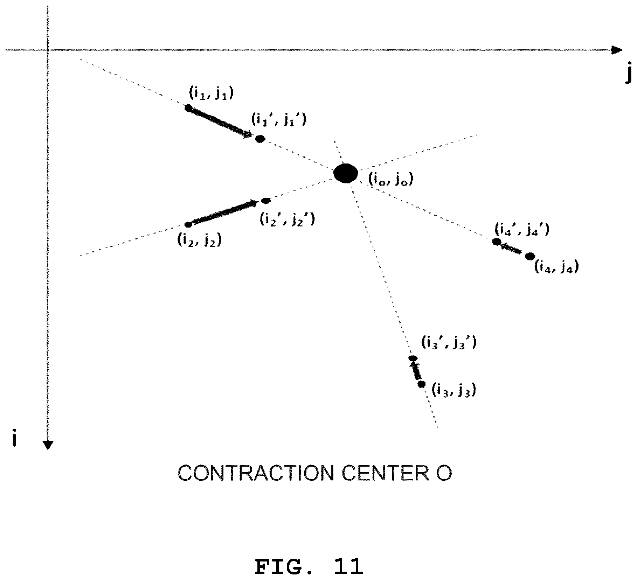

FIG. 11 is process of calculating a location of the contraction center O by the graph deriver 300a according to an embodiment of the present invention.

Referring to FIG. 11, the graph deriver 300a may calculate location data of the contraction center O based on the location data A before the procedure and the displacement data D even when the location of the contraction center O is not known. That is, in more detail, the graph deriver 300a sets the location data of the contraction center O corresponding to a location of a focus to which the energy-based medical device applies energy based on the landmark displacement data D, the location data of the contraction center O is a location of a point to which landmark points on the skin surface are commonly directed during skin coagulation according to the procedure, and a location of an intersection point of straight lines, which are obtained by points corresponding to the respective elements (i, j)ij of the location data A of the landmarks on the skin surface before the skin procedure each directed in a direction of an element iij vector of the landmark displacement data D, is set to (io, jo).

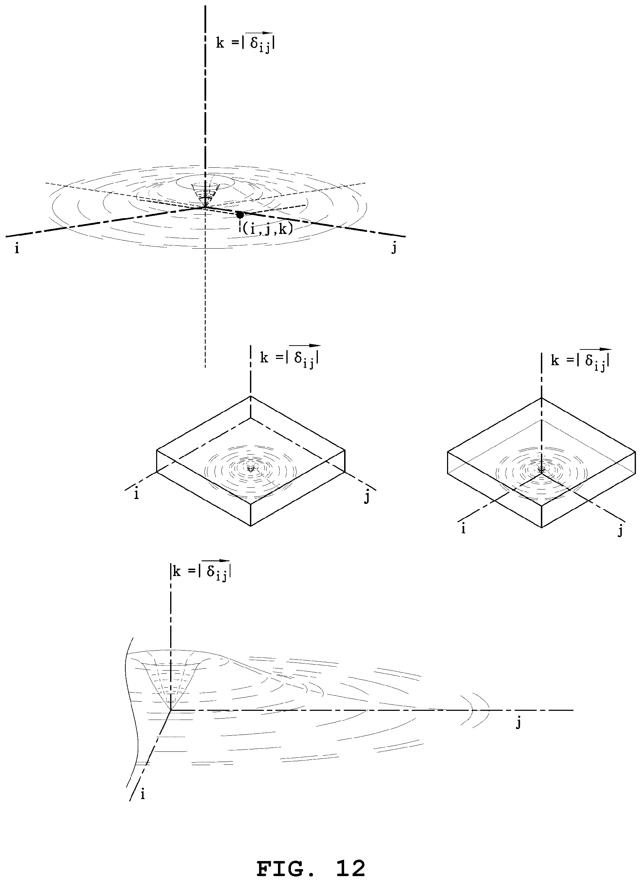

FIG. 12 is a graph of the graph deriver 300a according to an embodiment of the present invention.

Referring to FIG. 12, the graph deriver 300a may derive a graph of a distance (displacement) of each point moving toward the contraction center O for each point on a straight line passing through the contraction center O at a desired angle $\vec{\jmath}$ in the captured image before the procedure using the location data and the displacement data as input.

According to optimal embodiments of the present invention, the graph deriver 300a derives a displacement graph after the procedure with respect to a distance of each of landmarks on a straight line, which passes through the contraction center O in the skin procedure at the desired angle $\ominus$, from the contraction center O before the procedure, performs parallel translation by -io on the i-axis and -jo on the j-axis with respect to the contraction center O (io, jo, 0) in the skin procedure, and performs rotation by the angle $\ominus$ at which the contraction center O in the skin procedure is passed through on a k-axis, thereby deriving a graph of k with respect to i in an ik-plane (j = 0). That is, in more detail, in a three-dimensional (3D) Cartesian coordinate system obtained by adding the k-axis to the Cartesian coordinate system of the i-axis and the j-axis, a set of points [i, j, $|\vec{\delta}|$], where values corresponding to the i-axis and the j-axis are set to values i and j of an element [(i, j)ij] of the matrix A, and a value corresponding to the k-axis is set to the magnitude $|\vec{\delta}|$ of a vector element of the data D corresponding to the element [(i, j)ij] of matrix A, may be represented by a graph, which may be moved by -io on the i-axis and -jo on the j-axis to place a center of symmetry thereof on the k-axis, then rotated by the angle $\ominus$ around the k-axis, and then expressed as a graph of k with respect to i in the ik-plane (j = 0). In the process of deriving a graph, a location of a coagulation center O is not given, and a description has been given of a method capable of calculating a displacement distribution graph for all straight lines passing through the unknown coagulation center O. However, when the location of the coagulation center O is previously known, or only a displacement graph on one straight line is to be obtained, the corresponding step in the process of deriving a graph may be omitted.

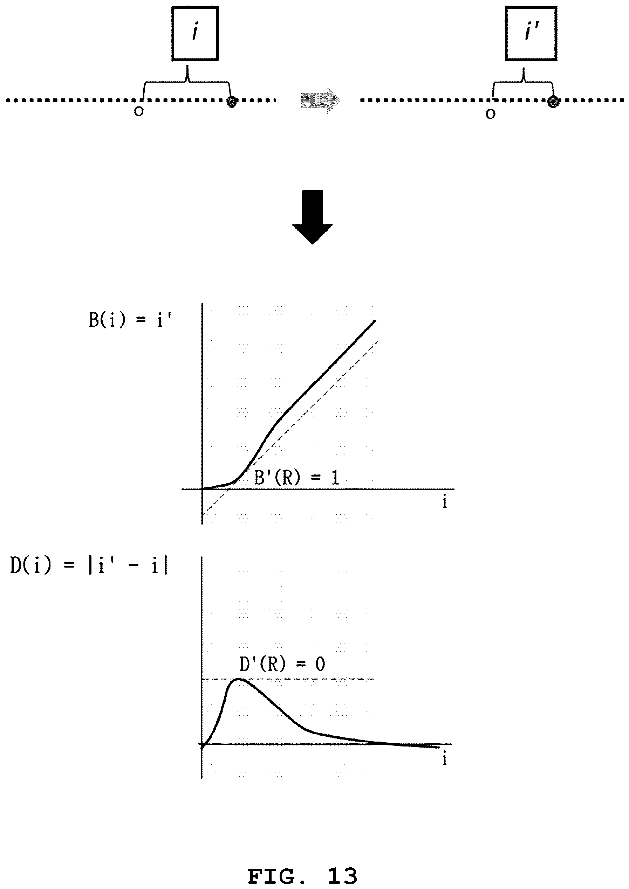

FIG. 13 is a process of calculating a size and shape of the coagulation center contraction zone by the boundary deriver 300b according to an embodiment of the present invention.

Referring to FIG. 13, the boundary deriver 300b derives a graph of a regression curve of a distance by which each point moves toward the contraction center O in the skin procedure for each location on the horizontal line $\vec{\jmath}$ = 0) passing through the contraction center O in the skin procedure, and calculates a distance from the contraction center O to the boundary R of the contraction zone from the captured image before the procedure based on the fact that the distance is close to a distance from the contraction center O to a point where a slope of a tangent becomes 0 on the regression curve. Therefore, it is possible to derive a regression curve for a scatter plot including discrete values obtained from actual observations.

As an embodiment of the present invention, the graph of the distance (displacement) by which each point moves toward the contraction center O for each point on the straight line passing through the contraction center O at the desired angle $\vec{\jmath}$ in the captured image before the procedure obtained by the graph deriver 300a regresses to a distribution curve D(i) having positive numbers as elements (Lognormal distribution, Chi-square distribution, F-distribution, and other distribution curves having positive numbers as elements). Thus, with respect to a distance (i) from the contraction center O to an element of the location data A before the procedure derived by the preprocessor 100, a graph of a distance (i') to an element of the location data B after the procedure corresponding to the element may be represented by B(i) = i - D(i). Therefore, the boundary deriver 300b may derive a size and shape of the contraction zone based on the fact that an i-coordinate at a point where a slope B'(i) of a tangent is 1 is close to a distance from the contraction center O to the boundary R of the contraction zone in the same direction on the curve B(i). Here, the graph of the distance (i') to the element of the location data B after the procedure corresponding to the element of the location data A before the procedure with respect to the distance (i) from the contraction center O to the element of the location data A before the procedure derived by the preprocessor 100 regresses to the curve B(i).

Further, in an embodiment of the present invention, when a cross section of the displacement graph derived by the graph deriver 300a regresses to a distribution curve having positive numbers as elements, the boundary deriver 300b may calculate the size and shape of the contraction zone based on the fact that a mode of the distribution, that is, an i-coordinate at a highest point where a slope of a tangent is 0 is close to a distance from the contraction center O to the boundary R of the contraction zone on a straight line passing through the contraction center O at an angle corresponding to the cross section. Since the distance from the contraction center O to the boundary R of the contraction zone may vary depending on the direction of the straight line passing through the contraction center O, the 3D graph derived by the graph deriver 300a may be rotated by a desired angle around the contraction center O to cause the boundary deriver 300b to calculate a distance to the boundary R of the contraction zone in all directions with respect to the contraction center O, which corresponds to obtaining the size and shape of the contraction zone.

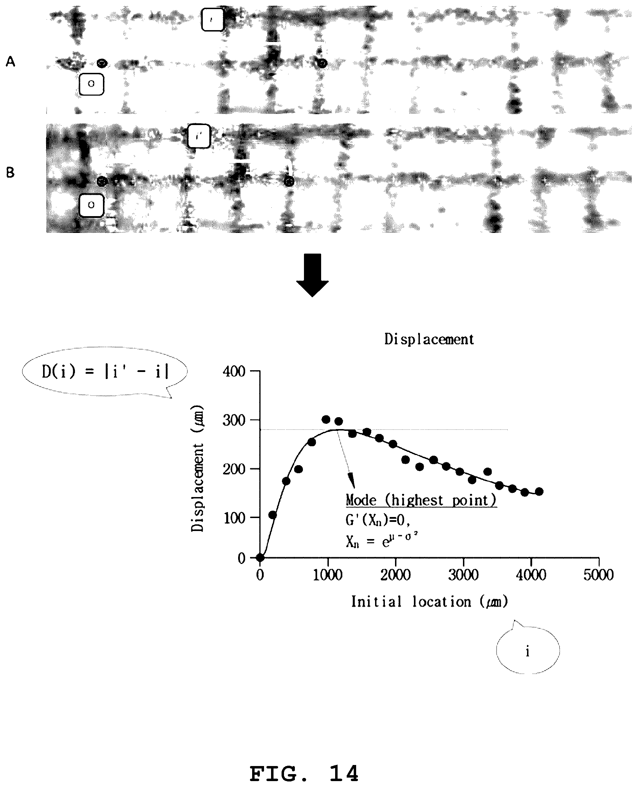

FIG. 14 is a calculation process when an angle of a straight line passing through a contraction center is 0 $\vec{\jmath}$ = 0) according to an embodiment of the present invention.

Referring to FIG. 14, the location data A before the procedure and the location data B after the procedure may be obtained based on landmark location data preprocessed by the preprocessor 100. Thereafter, the displacement calculator 200 calculates displacement D(i), which is a change in location data for each coagulation point of the skin, based on the location data acquired by the preprocessor 100. The location of the contraction center O is previously known or is obtained through calculation by the displacement calculator 200 as described in the embodiment of the graph deriver 300a, and then a graph is derived by the graph deriver 300a through this value. A cross section of a graph moved to the origin of a virtual coordinate system to calculate the size and shape of the contraction zone by the boundary deriver 300b regresses to a lognormal distribution with a regression coefficient of 0.6 or more ($R^2$ = 0.9562). Therefore, it may be determined that the scatter plot is not different from a population following the lognormal distribution of $D(i) = \dfrac{1}{i\sigma\sqrt{2\pi}}e^{\left\{-\frac{(\ln(i-\mu))^2}{2\sigma^2}\right\}}$. In the regressing displacement distribution curve D(i), a highest point corresponds to a mode, and a slope D'(i) of a tangent at the point is 0. In the lognormal distribution, a mode is $e^{\mu}$ (e being a natural constant, - being a mean, and - being a standard deviation), and thus a value i and a value i' satisfying D'(i) = 0 may be obtained by a probability density function equation. Therefore, by providing information on the shape and size of the contraction zone derived through the boundary deriver 300 to the operator or device, it is possible to implement an optimal state illustrated in the drawings of FIGs. 5 and 6, that is, a state in which boundaries of the contraction zones are in contact.

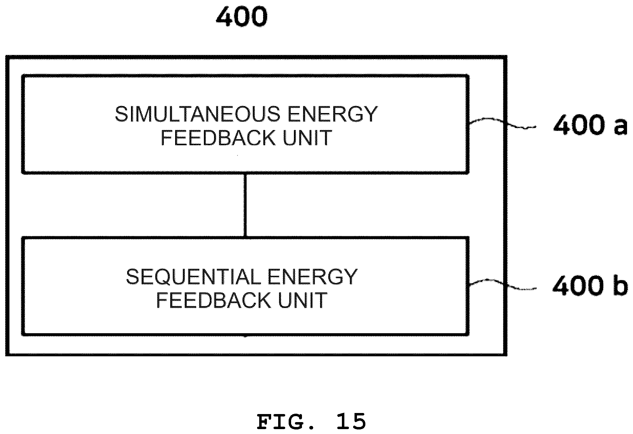

FIG. 15 is a configuration diagram of the procedure feedback unit 400 according to an embodiment of the present invention.

Referring to FIG. 15, the procedure feedback unit 400 includes a simultaneous energy feedback unit 400a and a sequential energy feedback unit 400b.

When coagulation spots are simultaneously generated through the energy-based medical device (simultaneous model), the simultaneous energy feedback unit 400a feeds back a minimum distance D preventing coagulation zones from overlapping each other. As an embodiment of the present invention, when a size of the contraction zone derived by the boundary deriver 300 is smaller than a fixed procedure distance of the energy-based medical device, the simultaneous energy feedback unit 400a feeds back high output energy value information to the energy-based medical device so that output of the energy-based medical device may be increased. Conversely, when the size of the contraction zone derived by the boundary deriver 300 is greater than the fixed procedure distance of the energy-based medical device, low output energy value information is fed back to the energy-based medical device so that output of the energy-based medical device may be decreased.

That is, in more detail, the simultaneous energy feedback unit 400a causes the energy-based medical device that promotes skin area contraction to generate several coagulation spots on the skin. Here, a type in which a plurality of coagulation spots is simultaneously generated is referred to as a simultaneous model. In the case of the simultaneous model, based on a calculation system of the present invention, a device capable of freely adjusting a distance between coagulation spots performs feedback so that output is increased when contraction zones are excessively small, and thus contraction zones are excessively far apart even when a distance between coagulation spots is minimized to an implementable distance, and conversely performs feedback so that output is decreased when contraction zones are excessively large, and thus contraction zones overlap each other even when the distance is maximized.

In addition, in the case of the simultaneous model, a device in which a distance between coagulation spots is fixed (or a device including discontinuous values for each step) performs feedback so that output is further increased when sizes of generated contraction zones are excessively small as a result of calculation, and thus a distance between boundaries is excessively large even in the case of an implementable minimum distance, and conversely performs feedback so that output is further decreased when contraction zones are excessively large, and thus it is determined that contraction zones overlap each other when several contraction zones are generated at a current interval.

Finally, in the case of a device in which a distance is adjusted to discontinuous values such as step 1/step 2/step 3, etc., feedback may be performed to increase or decrease the step of the distance or to decrease or increase the output.

When coagulation spots of the skin are sequentially generated through the energy-based medical device (sequential model), the sequential energy feedback unit 400b feeds back a minimum distance D' that prevents coagulation zones in (N-1)th to Nth rounds from overlapping, and feeds back a location of a coagulation spot to be generated in the Nth round based on the displacement graph derived by the boundary deriver 300 and information on a size and shape of a contraction zone based on one coagulation spot generated in the (N-1)th round. That is, in more detail, a type in which a plurality of coagulation spots is simultaneously generated is referred to as a simultaneous model, and in the case of the simultaneous model, a preferable location of a coagulation spot to be generated in an (N+1)th round is fed back based on a displacement graph obtained as a result of calculation based on a skin contraction aspect caused by one coagulation spot generated in the Nth round and information on a size and shape of a contraction zone.

According to the device factor calculation system based on skin surface displacement described above, there are the following effects. According to the device factor calculation system based on skin surface displacement described above, there are the following effects. First, it is possible to derive displacement of each point on a skin surface that contracts when one focal point on the skin is treated using an energy-based medical device. Second, it is possible to derive a location of a contraction center caused by the procedure based on photographs taken before and after the procedure. Third, it is possible to quantitatively express an aspect of skin contraction by the procedure based on the derived data. Fourth, it is possible to quantitatively express an aspect of skin contraction, which varies according to a patient undergoing the procedure and a part thereof and according to a type and output of the device, whenever the system is applied. Fifth, it is possible to generate data necessary for deriving an optimal next procedure location or procedure output during the procedure based on mathematical calculation rather than intuition of the operator. Sixth, it is possible to combine distribution information on a contraction aspect of skin in several parts in one procedure target to calculate and propose a 2D arrangement and order of several procedure points for realizing a change to desired appearance through manipulation of a location of each point on the skin.

Even though the detailed description of the present invention described above has been given with reference to preferred embodiments of the present invention, those skilled in the art or those having ordinary knowledge in the art may understand that the present invention may be variously modified and changed within the scope not departing from the spirit and technical scope of the present invention described in the claims to be described later.

[Industrial Applicability]

The device factor calculation system based on skin surface displacement may be installed in various energy-based medical devices such as fractional fine needle radiofrequency, fractional laser, and high-intensity focused ultrasound devices, and cosmetic effects may be maximized through automatic calculation of contraction of a skin plane and a calculated value.

**Claims**

1.  A skin procedure guidance and feedback system comprising:

    a photographing unit configured to photograph skin before and after a procedure by an energy-based medical device;
    a displacement calculator configured to derive landmark displacement data from landmark location data of the skin photographed by the photographing unit, and to derive location data of a contraction center O based on the landmark displacement data;
    a boundary deriver configured to derive a boundary of a contraction zone from the contraction center O in the skin procedure; and
    a procedure feedback unit configured to take result data derived by the boundary deriver as input to feed back optimal parameter information for the procedure.

2.  The skin procedure guidance and feedback system according to claim 1, wherein the energy-based medical device is configured to:

    apply energy to one focus of the skin to cause a local temperature rise, thereby generating a contraction zone based on the one focus of the skin, and
    include high-frequency, laser, and high-intensity focused ultrasound (HIFU) devices.

3.  The skin procedure guidance and feedback system according to claim 1, wherein the photographing unit photographs a skin surface before the procedure and a skin surface after the procedure based on the energy-based medical device.

4.  The skin procedure guidance and feedback system according to claim 1, wherein landmarks of the skin comprise dots or lines marked with pigment, and sweat glands, hair glands, sebaceous glands, pigmented lesions, moles, skin tumors, blood vessels, and wrinkle lines on a skin surface as reference points enabling tracking of positional change of a skin procedure target part before and after the procedure in the photographed images before and after the skin procedure through the energy-based medical device.

5.  The skin procedure guidance and feedback system according to claim 1, wherein the displacement calculator comprises:

    a preprocessor configured to convert location data A of landmarks before the skin procedure on a skin surface in an image of the skin surface before the skin procedure into $A[(i, j)ij]$, and to convert location data B of landmarks after the skin procedure on the skin surface in an image of the skin surface after the procedure into $B[(i', j')ij]$; and

a displacement data calculator configured to calculate, as the landmark displacement data D, a displacement vector reflecting a result of movement from location data before the skin procedure to location data after the procedure based on the landmark location data of the skin derived by the preprocessor.

6. The skin procedure guidance and feedback system according to claim 5, wherein the displacement data calculator represents the landmark displacement data D as [(i' - i, j' - j)ij], where i' and j' are a row i' and a column j' of B[(i', j')ij], and i and j are a row i and a column j of A[(i, j)ij].

7. The skin procedure guidance and feedback system according to claim 1, wherein the boundary deriver comprises:

a graph deriver configured to derive a displacement graph of each point moving toward the contraction center O for each location on a straight line passing through, at an angle $\mathbf{i}$, the contraction center O corresponding to a location of a focus to which the energy-based medical device applies energy based on the landmark displacement data; and
a boundary deriver configured to calculate a distance R from the contraction center O to the boundary of the contraction zone based on graph analysis result data derived by the graph deriver.

8. The skin procedure guidance and feedback system according to claim 7, wherein:

the graph deriver sets the location data of the contraction center O corresponding to a location of a focus to which the energy-based medical device applies energy based on the landmark displacement data D,
the location data of the contraction center O is a location of a point to which landmark points on a skin surface are commonly directed during skin coagulation according to the procedure, and
a location of an intersection point of straight lines, which are obtained by points corresponding to respective elements (i, j)ij of location data A of landmarks before the skin procedure on the skin surface each directed in a direction of an element iij vector of the landmark displacement data D, is set to (io, jo).

9. The skin procedure guidance and feedback system according to claim 7, wherein the graph deriver performs parallel translation by -io on an i-axis and - jo on a j-axis with respect to a contraction center O (io, jo, 0) in the skin procedure, and performs rotation by the angle $\Theta$ at which the contraction center O in the skin procedure is passed through on a k-axis, thereby deriving a graph of k with respect to i in an ik-plane (j = 0).

10. The skin procedure guidance and feedback system according to claim 7, wherein the boundary deriver is configured to:

derive a graph of a regression curve of a distance by which each point moves toward the contraction center O in the skin procedure for each location on a horizontal line $\Theta$ = 0) passing through the contraction center O in the skin procedure, and
calculate a distance from the contraction center O to the boundary R of the contraction zone from the photographed image before the procedure based on a fact that the distance is close to a distance from the contraction center O to a point where a slope of a tangent becomes 0 on the regression curve.

11. The skin procedure guidance and feedback system according to claim 1, wherein the procedure feedback unit comprises a simultaneous energy feedback unit configured to feed back a minimum distance D preventing coagulation zones from overlapping each other when coagulation spots are simultaneously generated through the energy-based medical device (simultaneous model).

12. The skin procedure guidance and feedback system according to claim 11, wherein, when a size of the contraction zone derived by the boundary deriver is smaller than a fixed procedure distance of the energy-based medical device, the simultaneous energy feedback unit feeds back high output energy value information to the energy-based medical device so that output of the energy-based medical device is allowed to be increased.

13. The skin procedure guidance and feedback system according to claim 11, wherein, when a size of the contraction zone derived by the boundary deriver is greater than a fixed procedure distance of the energy-based medical device, the simultaneous energy feedback unit feeds back low output energy value information to the energy-based medical device so that output of the energy-based medical device is allowed to be decreased.

14. The skin procedure guidance and feedback system according to claim 1, wherein the procedure feedback unit comprises a sequential energy feedback unit configured to feed back a minimum distance D' that prevents coagulation zones in (N-1)th to Nth rounds from overlapping when coagulation spots on the skin are sequentially generated through the energy-based medical device (sequential model).

15. The skin procedure guidance and feedback system according to claim 14, wherein the sequential energy feedback unit feeds back a location of a coagulation spot to be generated in the Nth round based on a displacement graph derived by the boundary deriver and information on a size and shape of a contraction zone based on one coagulation spot generated in the (N-1)th round.

PHOTOGRAPHING UNIT — **100**

DISPLACEMENT CALCULATOR — **200**

BOUNDARY DERIVER — **300**

PROCEDURE FEEDBACK UNIT — **400**

FIG. 1

h = x $\mu$m

FIG. 2

FIG. 3

Contraction zone

Compensatory dilatation zone

Compensato ry dilatation

Contraction

Compensato ry dilatation

FIG. 4

(CASE 1) WHEN PLURALITY OF COAGULATION SPOTS IS SEQUENTIALLY GENERATED

## (Linear, sequential model)

(A) WHEN BOUNDARIES OF TWO ADJACENT COAGULATION RANGES ARE IN CONTACT

AIM AT COAGULATION SPOT 1

GENERATION OF COAGULATION SPOT 1

AIM AT COAGULATION SPOT 2

GENERATION OF COAGULATION SPOT 2

(B) WHEN BOUNDARIES OF TWO ADJACENT COAGULATION RANGES ARE SEPARATED FROM EACH OTHER

AIM AT COAGULATION SPOT 1

GENERATION OF COAGULATION SPOT 1

AIM AT COAGULATION SPOT 2

GENERATION OF COAGULATION SPOT 2

COMPENSATORY DILATATION BETWEEN COAGULATION SPOTS

AREA REDUCTION EFFICIENCY DETERIORATES

(C) WHEN TWO ADJACENT COAGULATION RANGES OVERLAP EACH OTHER

AIM AT COAGULATION SPOT 1

GENERATION OF COAGULATION SPOT 1

AIM AT COAGULATION SPOT 2

GENERATION OF COAGULATION SPOT 2

COAGULATION ZONES OVERLAP

RISK DUE TO OVER-ENERGY TRANSFER

# FIG. 5

(CASE 2) WHEN PLURALITY OF COAGULATION SPOTS IS GENERATED AT THE SAME TIME

## (Linear, simultaneous model)

FIG. 6

**200**

```
┌─────────────────────────────────────────────────┐
│  ┌───────────────────────────────────────────┐  │
│  │                                           │  │
│  │            PREPROCESSOR                    │  │ ～ **200 a**
│  │                                           │  │
│  └───────────────────────────────────────────┘  │
│                       │                          │
│  ┌───────────────────────────────────────────┐  │
│  │                                           │  │
│  │     DISPLACEMENT DATA CALCULATOR          │  │ ～ **200 b**
│  │                                           │  │
│  └───────────────────────────────────────────┘  │
└─────────────────────────────────────────────────┘
```

FIG. 7

BEFORE
PROCEDURE

AFTER
PROCEDURE

FIG. 8

$$\vec{\delta}_{ij}$$

$(i, j)_{ij}$       $(i', j')_{ij}$

DISPLACEMENT

FIG. 9

**300**

- GRAPH DERIVER — **300 a**
- BOUNDARY CALCULATOR — **300 b**

FIG. 10

CONTRACTION CENTER O

FIG. 11

FIG. 12

$B(i) = i'$

$B'(R) = 1$

$D(i) = |i' - i|$

$D'(R) = 0$

FIG. 13

**FIG. 14**

**400**

```
┌─────────────────────────────────────────────┐
│  ┌───────────────────────────────────┐       │
│  │      SIMULTANEOUS ENERGY          │       │  ⌇ 400 a
│  │       FEEDBACK UNIT               │       │
│  └───────────────────────────────────┘       │
│                    │                          │
│  ┌───────────────────────────────────┐       │
│  │       SEQUENTIAL ENERGY           │       │  ⌇ 400 b
│  │        FEEDBACK UNIT              │       │
│  └───────────────────────────────────┘       │
└─────────────────────────────────────────────┘
```

FIG. 15

## INTERNATIONAL SEARCH REPORT

| | International application No. |
|---|---|
| | **PCT/KR2022/017759** |

### A. CLASSIFICATION OF SUBJECT MATTER

**A61B 5/00**(2006.01)i; **G06T 7/00**(2006.01)i; **G06T 7/60**(2006.01)i

According to International Patent Classification (IPC) or to both national classification and IPC

### B. FIELDS SEARCHED

Minimum documentation searched (classification system followed by classification symbols)

A61B 5/00(2006.01); A61B 5/11(2006.01); A61B 8/00(2006.01); A61B 8/08(2006.01); A61N 5/06(2006.01); A61N 5/067(2006.01); G06T 7/00(2006.01); G06T 7/62(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부(skin), 레이저(laser), 이미지(image), 랜드마크(landmark), 변위(displacement), 수축(contraction), 경계(boundary), 피드백(feedback)

### C. DOCUMENTS CONSIDERED TO BE RELEVANT

| Category* | Citation of document, with indication, where appropriate, of the relevant passages | Relevant to claim No. |
|---|---|---|
| A | KR 10-2015-0135776 A (SHISEIDO COMPANY, LIMITED) 03 December 2015 (2015-12-03)<br>See claim 1. | 1-15 |
| A | KR 10-2188612 B1 (KOLMAR KOREA CO., LTD. et al.) 09 December 2020 (2020-12-09)<br>See claim 2. | 1-15 |
| A | US 2019-0099159 A1 (SIEMENS HEALTHCARE GMBH et al.) 04 April 2019 (2019-04-04)<br>See entire document. | 1-15 |
| A | JP 2021-523765 A (KONINKLIJKE PHILIPS N.V.) 09 September 2021 (2021-09-09)<br>See entire document. | 1-15 |
| A | JP 2016-512783 A (DERMA DREAM GROUP LTD.) 09 May 2016 (2016-05-09)<br>See entire document. | 1-15 |

☐ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **04 May 2023** | **04 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2022/017759**

| Patent document cited in search report | | | Publication date (day/month/year) | Patent family member(s) | | | Publication date (day/month/year) |
|---|---|---|---|---|---|---|---|
| KR | 10-2015-0135776 | A | 03 December 2015 | CN | 105101874 | A | 25 November 2015 |
| | | | | CN | 105101874 | B | 15 August 2017 |
| | | | | EP | 2979634 | A1 | 03 February 2016 |
| | | | | HK | 1211452 | A1 | 27 May 2016 |
| | | | | JP | 2014-193197 | A | 09 October 2014 |
| | | | | JP | 6251489 | B2 | 20 December 2017 |
| | | | | TW | 201446215 | A | 16 December 2014 |
| | | | | TW | I612938 | B | 01 February 2018 |
| | | | | US | 2016-0063312 | A1 | 03 March 2016 |
| | | | | US | 9607208 | B2 | 28 March 2017 |
| | | | | WO | 2014-156461 | A1 | 02 October 2014 |
| KR | 10-2188612 | B1 | 09 December 2020 | None | | | |
| US | 2019-0099159 | A1 | 04 April 2019 | CN | 109584350 | A | 05 April 2019 |
| | | | | DE | 102018216296 | A1 | 04 April 2019 |
| | | | | KR | 10-2019-0038448 | A | 08 April 2019 |
| | | | | KR | 10-2269467 | B1 | 24 June 2021 |
| | | | | US | 10660613 | B2 | 26 May 2020 |
| JP | 2021-523765 | A | 09 September 2021 | CN | 112074237 | A | 11 December 2020 |
| | | | | EP | 3787518 | A1 | 10 March 2021 |
| | | | | US | 2021-0106312 | A1 | 15 April 2021 |
| | | | | WO | 2019-211175 | A1 | 07 November 2019 |
| JP | 2016-512783 | A | 09 May 2016 | AU | 2014-233770 | A1 | 15 October 2015 |
| | | | | CN | 105407824 | A | 16 March 2016 |
| | | | | EP | 2976035 | A1 | 27 January 2016 |
| | | | | HK | 1222528 | A1 | 07 July 2017 |
| | | | | KR | 10-2015-0133800 | A | 30 November 2015 |
| | | | | US | 2016-0045762 | A1 | 18 February 2016 |
| | | | | WO | 2014-147624 | A1 | 25 September 2014 |

Form PCT/ISA/210 (patent family annex) (July 2022)